# EUROPÄISCHE PATENTSCHRIFT

(11) **EP 1 462 149 B1**
(45) Veröffentlichungstag und Bekanntmachung des Hinweises auf die Patenterteilung: **07.05.2008**
(21) Anmeldenummer: 04006037.8
(22) Anmeldetag: 13.03.2004
(51) Int. Cl.: A61P 29/00, A61K 31/4152, A61K 9/20

(54) **Verwendung von Phenazon zur Behandlung von Migräne**
Use of phenazon for the treatment of migraine
Utilisation du phenazon pour le traitement du migraine

(30) Priorität: 26.03.2003 DE 10313446
(43) Veröffentlichungstag der Anmeldung: 29.09.2004
(73) Patentinhaber: Krewel Meuselbach GmbH, 53783 Eitorf (DE)
(72) Erfinder: Schierstedt, Dieter, Dr., 53757 St. Augustin (DE)
(74) Vertreter: Jönsson, Hans-Peter

(56) Entgegenhaltungen:
- EP-B- 0 876 141
- VOIGT, R. ET AL.: "Beiträge zur Erhöhung der Liberationsgeschwindigkeit von Propyphenazon aus peroralen festen Arzneiformen" DIE PHARMAZIE, Bd. 40, Nr. 1, Januar 1985 (1985-01), Seiten 39-44, XP001189632

## Beschreibung

Die Erfindung betrifft die Verwendung von Phenazon als alleinigen Wirkstoff zur Herstellung eines Medikaments gegen Migräne mit Highspeed-Galenik.

Unter den Begriff "Migräne" fallen anfallartige, oftmals pulsierende Kopfschmerzen, die gemäß Klassifikation nach der International Headache Society (IHS) mit oder ohne Aura auftreten können. Migräne kann typischerweise von Hörstörungen, visuellen Symptomen oder neurologischen Ausfällen begleitet sein sowie von einer gesteigerten Empfindlichkeit gegenüber Licht oder Lärm.

Zur Behandlung akuter Anfälle von Migräne (Kopfschmerzphase mit und ohne Aura) werden im Stand der Technik vielfach Analgetika wie Ibuprofen, Acetylsalicylsäure sowie Paracetamol verabreicht. Mit den genannten Wirkstoffen können zum Teil erhebliche Nebenwirkungen im Magen-Darmtrakt und/oder in der Leber verbunden sein.

Phenazon ist ein Wirkstoff aus der Pyrazolonreihe, der die für diese Substanzklasse typischen seltenen Nebenwirkungen wie beispielsweise allergische Schockreaktionen und Agranolocystose nicht aufweist. Als Basis-Analgetikum für Migräne- und Kopfschmerzbeschwerden ist Phenazon daher grundsätzlich potentiell geeignet, - wurde allerdings bisher für diese Indikation kaum eingesetzt. Dies liegt zum einen an dem verbreiteten Vorurteil gegen Wirkstoffe aus der Pyrazolonreihe, zum anderen in den physikalischen Eigenschaften des Wirkstoffs Phenazon begründet, der mit herkömmlichen galenischen Formulierungen keine ausreichende Freisetzungsgeschwindigkeit in vivo erzielen kann.

EP-B-876 141 beschreibt schnell freisetzende Zubereitungen nichtsteroidaler Analgetika wie z.B. Phenazon. Die Zubereitungen enthalten Homo- als auch Copolymere des N-Vinylpyrrolidons sowie Carbonate. Die nach 30 Minuten gemessene Freisetzung beträgt bis zu 90 Gew.-%.

Eine unabdingbare Voraussetzung für eine erfolgreiche Behandlung eines Migräne- bzw. Kopfschmerzanfalls ist allerdings eine schnelle Freisetzung und Resorption des Wirkstoffs. Mit herkömmlichen galenischen Zubereitungen von Phenazon ist dies nicht gewährleistet, da Phenazon aufgrund seiner osmotischen Eigenschaften mit herkömmlichen Tabletten-Sprengmitteln in üblicher Konzentration nicht verträglich ist.

Typische Konzentrationen für Sprengmittel, beispielsweise vom Polyvidontyp (Polydon^{®} CL, Kollidon^{®} CL-M und Cospovidon^{®} M) betragen 1 bis 2 Gew.-%, bezogen auf das fertige Arzneimittel, in Einzelfällen sind auch Konzentrationen bis zu 5 Gew.-%, bezogen auf das Arzneimittel üblich.

In diesen Konzentrationen behindert der außerordentlich stark wasserlösliche Wirkstoff Phenazon die Wirkung dieser Hilfsstoffe osmotisch so stark, dass die Freisetzung sogar erheblich langsamer erfolgt, als in Sprengmittel-freien wasserlöslichen Rezepturen.

Solche reinen wasserlöslichen Rezepturen setzen den Wirkstoff jedoch ebenfalls nicht ausreichend schnell frei, um einen Migräne-Kopfschmerz-Anfall erfolgreich behandeln zu können.

Aufgabe der vorliegenden Erfindung war daher, Phenazon-Formulierungen bereitzustellen, die die speziellen Anforderungen einer Highspeed-Galenik erfüllen und so die Herstellung eines wirksamen Migränemedikamentes auf Phenazon-Basis als alleinigen Wirkstoff ermöglichen.

Die Aufgabe wird erfindungsgemäß gelöst durch die Verwendung von Phenazon als alleinigen Wirkstoff zur Herstellung eines Medikaments gegen Migräne, das dadurch gekennzeichnet ist, dass das Medikament in vivo eine Phenazon-Freisetzungsrate von mindestens 95 % innerhalb von 15 min. aufweist, die in einer Blattrührerapparatur bestimmt wird, wobei die Temperatur des Lösungsmedium 37 °C, und die Umdrehungsgeschwindigkeit des Rührers 100 U/min beträgt, und zu Beginn der Untersuchung jede Tablette in 900 ml künstlichen Magensaft (0,1 mol/l HCl) gegeben wird, und zu dem vorbestimmten Zeitpunkt im Lösungsmedium befindliche freigesetzte Wirkstoffmenge (Phenazon) mittels HPLC bestimmt wird.

Dabei bezieht sich die Verwendung von Phenazon auf eine übliche Menge, das heißt eine Menge, die ausreicht, die Migräne-Beschwerden zu lindern.

Freisetzungsraten aus Arzneimittelzubereitungen im Sinne der Erfindung werden nach DAB10 (Deutsches Arzneimittelbuch, 10. Auflage) in einer Blattrührerapparatur bestimmt. Die Temperatur des Lösungsmediums beträgt 37 °C, und die Umdrehungsgeschwindigkeit des Rührers beträgt 100 U/min. Zu Beginn der Untersuchung wird jede Tablette in 900 ml künstlichen Magensaft (0,1 mol/l HCl) gegeben. Die zu dem vorbestimmten Zeitpunkt im Lösungsmedium befindliche freigesetzte Wirkstoffmenge (Phenazon) wird mittels HPLC bestimmt.

Bei einer Freisetzungsrate von mindestens 95 % innerhalb von 15 min, insbesondere bei einer Freisetzungsrate von mindestens 95 % innerhalb von 10 min, ist gewährleistet, dass der Wirkstoff Phenazon in einem Schub die gewünschte Wirkung entfalten kann und die Migräne-Beschwerden ohne bzw. mit Aura wirkungsvoll lindert.

In einer weiteren Ausführungsform der Erfindung wird die gestellte Aufgabe dadurch gelöst, dass das Medikament neben Phenazon als alleinigem Wirkstoff 11 bis 30 Gew.-% eines Sprengmittels enthält.

Überraschend wurde gefunden, dass Phenazon, das mit einem Vielfachen der herkömmlichen Menge an Sprengmitteln versetzt wurde, eine Highspeed-galenische Formulierung ermöglicht, mit der die nötigen Freisetzungsraten erzielt werden können. Beste Freisetzungsraten werden erzielt, wenn das Medikament 12 bis 20 Gew.-%, besonders bevorzugt 13 bis 15 Gew.-% eines Sprengmittels enthält.

In einer weiteren Ausführungsform beträgt das Gewichts-Verhältnis von Sprengmittel zu Phenazon 5 bis 200 %, bevorzugt 15 bis 50 %, insbesondere 17 bis 30 %, ganz besonders bevorzugt 18 bis 25 %, wodurch eine optimale Freisetzungsrate erzielt wird.

Besonders bevorzugte Sprengmittel, die erfindungsgemäß eingesetzt werden können, sind quervernetztes Polyvidon, insbesondere Cross-Povidon und/oder Kollidon^{®} CL.

Als Sprengmittel können bevorzugt auch alternativ oder in Kombination Hydrogencarbonate und/oder Carbonate enthalten sein.

Hierdurch ist insbesondere die Herstellung von Brausetabletten, Brausepulver sowie Brausegranulat möglich.

Zur optimalen Entfaltung der schmerzlindernden Wirkung enthalten die erfindungsgemäßen Medikamente bevorzugt 500 bis 1000 mg Phenazon.

In einer weiteren Ausführungsform der Erfindung können neben den genannten Inhaltsstoffen noch ein oder mehrere weitere Hilfsstoffe enthalten sein, ausgewählt aus der Gruppe Avicel, Aerosil, Magnesiumstearat, Lactose, Zucker, Süßstoffe, Aromastoffe, Farbstoffe, Vitamin C, sowie Füllstoffe.

Die erfindungsgemäßen Medikamente werden bevorzugt in Form von Tabletten, Brausetabletten, Brausepulver, Brausegranulat, Dragees, Filmtabletten und/oder Kapseln bereitgestellt.

### Ausführungsbeispiele:

### Beispiel 1:

| Rohstoffbezeichnung | 1 Tablette enthält |
|---|---|
| Phenazon mikro.Ph.Eur. | 500,000 mg |
| Aqua dem. | (35,000 mg) |
| Kollidon^{®} CL Ph.Eur. | 110,000 mg |
| Avicel^{®} 102 Ph.Eur. | 95,000 mg |
| Aerosil^{®} gepresst Ph.Eur. | 31,000 mg |
| Magnesiumstearat Ph.Eur. | 3,000 mg |
| Gesamt: | 739,000 mg |

Aus den genannten Bestandteilen wurde durch Direktpressung eine Tablette hergestellt. Die Freisetzungsrate des Phenazons wurde bestimmt und betrug nach 10 min. 96 %.

### Beispiel 2:

Mit einer erfindungsgemäßen Rezeptur ist es in einer klinischen Studie erstmals gelungen (Doppelblind ./. Placebo) zu beweisen, dass Phenazon bei Migräne eine wirkungsvolle Alternative zu den bisher häufig gebraucht Analgetika wie Ibuprofen, Acetylsalicylsäure oder Paracetamol darstellt.

Der Vorteil von Phenazon als alleiniger Wirkstoff gegenüber den letztgenannten besteht in geringeren Nebenwirkungen im Magen-DarmTrakt bzw. bezüglich der Leber. 240 Patienten, die die IHS-Kriterien für Migräne mit oder ohne Aura erfüllten wurden für die Studie ausgewählt. Gemäß einem Doppelblind/Placebo-kontrollierten Studiendesign wurden jeweils 120 zufällig ausgewählte Patienten entweder mit dem Wirkstoff (gemäß Beispiel 1) oder Placebo behandelt. Kopfschmerzintensität, begleitende Symptome, Nebenwirkungen und die Einnahme von Rescue-Medication wurde in einem Tagebuch verzeichnet.

### Ergebnisse:

208 Patienten haben die Studie gemäß der Versuchsbeschreibung beendet (105 Phenazon- und 103 Placebopatienten). Die Responderrate 2 Std. nach der Dosisgabe (Linderung von mäßigen/schweren Kopfschmerzen zu milden/keinen Kopfschmerzen) war in der Phenazongruppe signifikant höher (48,6 % gegenüber 27,2 %), mehr Patienten waren völlig frei von Kopfschmerz (27,6 % gegenüber 13,6 %), mehr Patienten berichteten Linderung von Übelkeit, Lichtempfindlichkeit sowie Lärmempfindlichkeit und weniger Patienten benötigten Rescue-Medikation (38,1 % gegenüber 69,9 %). 11,4 % der Patienten der Phenazongruppe berichteten von Nebenwirkungen, im Vergleich zu 5,8 % der Placebogruppe. Diese bezogen sich größtenteils auf den Magen-Darm-Trakt (Übelkeit, trockener Mund, Unterleibsschmerzen). Alle Nebenwirkungen waren von kurzer Dauer.

Die erfindungsgemäßen Phenazonpräparate haben sich in der Studie als wirksame und verträgliche Medikamente für die Behandlung akuter Migräne erwiesen.

## Patentansprüche

1. Verwendung von Phenazon als alleinigen Wirkstoff zur Herstellung eines Medikaments gegen Migräne, **dadurch gekennzeichnet, dass** das Medikament in vivo eine Phenazon-Freisetzungsrate von mindestens 95 % innerhalb von 15 min. aufweist, die in einer Blattrührerapparatur bestimmt wird, wobei die Temperatur des Lösungsmedium 37 °C, und die Umdrehungsgeschwindigkeit des Rührers 100 U/min beträgt, und zu Beginn der Untersuchung jede Tablette in 900 ml künstlichen Magensaft (0,1 mol/l HCl) gegeben wird, und zu dem vorbestimmten Zeitpunkt im Lösungsmedium befindliche freigesetzte Wirkstoffmenge (Phenazon) mittels HPLC bestimmt wird.

2. Verwendung gemäß Anspruch 1, **dadurch gekennzeichnet, dass** die Phenazon-Freisetzungsrate mindestens 95 % innerhalb von 10 min. beträgt.

3. Verwendung von Phenazon als alleinigen Wirkstoff zur Herstellung eines Medikaments gegen Migräne, **dadurch gekennzeichnet, dass** es 11 bis 30 Gew.-% eines Sprengmittels enthält.

4. Verwendung gemäß Anspruch 3, **dadurch gekennzeichnet, dass** es 12 bis 20 Gew.-%, insbesondere 13 bis 15 Gew.-% eines Sprengmittels enthält.

5. Verwendung von Phenazon als alleinigen Wirkstoff zur Herstellung eines Medikaments gegen Migräne, **dadurch gekennzeichnet, dass** das Gewichts-Verhältnis von Sprengmittel zu Phenazon 5 bis 200 % beträgt.

6. Verwendung gemäß Anspruch 5, **dadurch gekennzeichnet, dass** das Gewichtsverhältnis von Sprengmittel zu Phenazon 15 bis 50 %, insbesondere 17 bis 30 %, ganz besonders 18 bis 25 % beträgt.

7. Verwendung gemäß einem oder mehreren der Ansprüche 1 bis 6, **dadurch gekennzeichnet, dass** das Medikament als Sprengmittel quervernetztes Polyvidon enthält.

8. Verwendung gemäß einem oder mehreren der Ansprüche 1 bis 6, **dadurch gekennzeichnet, dass** das Medikament als Sprengmittel Hydrogencarbonate und/oder Carbonate enthält

9. Verwendung gemäß einem oder mehreren der Ansprüche 1 bis 8, **dadurch gekennzeichnet, dass** es 500 bis 1000 mg Phenazon enthält.

10. Verwendung gemäß einem oder mehreren der Ansprüche 1 bis 9, **dadurch gekennzeichnet, dass** das Medikament einen oder mehrere weitere Hilfsstoffe, ausgewählt aus der Gruppe Avicel, Aerosil, Magnesiumstearat, Lactose, Zucker, Süßstoffe, Aromastoffe, Farbstoffe, Vitamin C sowie Füllstoffe enthält.

11. Verwendung gemäß einem der Ansprüche 1 bis 10 in Form von Tabletten, Brausetabletten, Brausepulver, Brausegranulat, Dragees, Filmtabletten und/oder Kapseln.

## Claims

1. Use of phenazone as a sole active ingredient for preparing a medicament against migraine, **characterized in that** said medicament has a phenazone release rate of at least 95% within 15 min in vivo as determined in a paddle agitator apparatus wherein the temperature of the solvent is 37 °C, and the revolutions per minute of the agitator is 100 rpm, and at the beginning of the examination, each tablet is added to 900 ml of artificial gastric juice (0.1 mol/l HCl), and the quantity of active ingredient (phenazone) released that is present in the solvent at a predetermined time is determined by HPLC.

2. The use according to claim 1, **characterized in that** said phenazone release rate is at least 95% within 10 min.

3. Use of phenazone as a sole active ingredient for preparing a medicament against migraine, **characterized by** containing from 11 to 30% by weight of a disintegrant.

4. The use according to claim 3, **characterized by** containing from 12 to 20% by weight, especially from 13 to 15% by weight, of a disintegrant.

5. Use of phenazone as a sole active ingredient for preparing a medicament against migraine, **characterized in that** the weight ratio of disintegrant to phenazone is from 5 to 200%.

6. The use according to claim 5, **characterized in that** the weight ratio of disintegrant to phenazone is from 15 to 50%, especially from 17 to 30%, more particularly from 18 to 25%.

7. The use according to one or more of claims 1 to 6, **characterized in that** said medicament contains cross-linked polyvidone as said disintegrant.

8. The use according to one or more of claims 1 to 6, **characterized in that** said medicament contains hydrogencarbonates and/or carbonates as said disintegrant.

9. The use according to one or more of claims 1 to 8, **characterized by** containing from 500 to 1000 mg of phenazone.

10. The use according to one or more of claims 1 to 9, **characterized in that** said medicament contains one or more further auxiliary agents selected from the group consisting of Avicel, Aerosil, magnesium stearate, lactose, sugar, sweetening agents, flavoring agents, coloring agents, vitamin C as well as fillers.

11. The use according to any of claims 1 to 10 in the form of tablets, effervescent tablets, effervescent powder, effervescent granules, coated tablets, film tablets and/or capsules.

## Revendications

1. Utilisation de phénazone en tant que seule substance active pour préparer un médicament contre la migraine, **caractérisée en ce que** ledit médicament a un taux de libération de phénazone d'au moins 95% dans 15 min in vivo, déterminé dans un appareil d'agitateur à palettes, la température du solvant étant de 37 °C, et la vitesse de rotation dudit agitateur étant de 100 révolutions par minute, et au début de l'examen, chaque tablette est ajoutée à 900 ml de suc gastrique artificiel (0,1 mol/l de HCl), et la quantité de substance active (phénazone) libérée présente dans le solvant à l'instant prédéterminé est déterminée par HPLC.

2. Utilisation selon la revendication 1, **caractérisée en ce que** ledit taux de libération de phénazone est au moins 95% dans 10 min.

3. Utilisation de phénazone en tant que seule substance active pour préparer un médicament contre la migraine, **caractérisée en ce qu'**il contient de 11 à 30% en poids d'un désintégrateur.

4. Utilisation selon la revendication 3, **caractérisée en ce qu'**il contient de 12 à 20% en poids, notamment de 13 à 15% en poids, d'un désintégrateur.

5. Utilisation de phénazone en tant que seule substance active pour préparer un médicament contre la migraine, **caractérisée en ce que** le rapport en poids entre le désintégrateur et la phénazone est de 5 à 200%.

6. Utilisation selon la revendication 5, **caractérisée en ce que** le rapport en poids entre le désintégrateur et la phénazone est de 15 à 50%, notamment de 17 à 30%, plus particulièrement de 18 à 25%.

7. Utilisation selon l'une ou plusieurs des revendications 1 à 6, **caractérisée en ce que** le médicament contient de la polyvidone réticulée en tant que désintégrateur.

8. Utilisation selon l'une ou plusieurs des revendications 1 à 6, **caractérisée en ce que** le médicament contient des hydrogènocarbonates et/ou des carbonates en tant que désintégrateurs.

9. Utilisation selon l'une ou plusieurs des revendications 1 à 8, **caractérisée en ce qu'**il contient de 500 à 1000 mg de phénazone.

10. Utilisation selon l'une ou plusieurs des revendications 1 à 9, **caractérisée en ce que** ledit médicament contient un ou plusieurs autres agents auxiliaires choisis dans le groupe comprenant l'Avicel, l'Aerosil, le stéarate de magnésium, le lactose, le sucre, les édulcorants, les arômes, les colorants, la vitamine C et les charges.

11. Utilisation selon l'une quelconque des revendications 1 à 10 sous la forme de tablettes, tablettes effervescentes, poudre effervescente, granules effervescents, dragées, tablettes de film et/ou capsules.
